# EUROPÄISCHE PATENTSCHRIFT

(11) **EP 3 285 732 B1**
(45) Veröffentlichungstag und Bekanntmachung des Hinweises auf die Patenterteilung: **20.11.2019**
(21) Anmeldenummer: 16719284.8
(22) Anmeldetag: 14.04.2016
(51) Int. Cl.: A61Q 19/10, A61K 8/36, A61K 8/37, C07D 493/04, C11D 3/00, C11D 1/00

(54) **ISOSORBIDDIESTER ALS PERLGLANZMITTEL UND TRÜBUNGSMITTEL**
ISOSORBIDE DIESTERS AS PEARLESCENT AGENT AND CLOUDING AGENT
DIESTERS D'ISOSORBIDE EN TANT QU'AGENT NACRÉ ET OPACIFIANT

(30) Priorität: 24.04.2015 EP 15165053
(43) Veröffentlichungstag der Anmeldung: 28.02.2018
(73) Patentinhaber: BASF SE, 67056 Ludwigshafen (DE)
(72) Erfinder: STOER, Claudia, 40597 Düsseldorf (DE); WEISSENEGGER, Markus, 40627 Düsseldorf (DE); NIEENDICK, Claus, 47807 Krefeld (DE); WINZEK, Mirella, 52445 Titz (DE)
(74) Vertreter: BASF IP Association
(86) Internationale Anmeldenummer: PCT/EP2016/058217
(87) Internationale Veröffentlichungsnummer: WO 2016/169833

(56) Entgegenhaltungen:
- EP-A1- 2 239 315
- WO-A1-01/83488
- WO-A1-2013/041388
- WO-A1-2016/005239
- US-A1- 2014 322 151
- JASINSKI WLODZIMIERZ ET AL: "Preparation of monoesters of 1,4-3,6-dianhydro-D-sorbitol and higher fatty acids", PRACE NAUKOWE INSTYTUTU TECHNOLOGII ORGANICZNEJ I TWORZYW SZTUCZNYCH POLITECHNIKI WROCLAWSKIEJ,, Nr. 12, 1. Januar 1973 (1973-01-01), Seiten 31-74, XP009185418, ISSN: 0370-0879

## Beschreibung

Die vorliegende Erfindung betrifft Zusammensetzungen, enthaltend mindestens einen Isosorbiddiester, mindestens einen Isosorbidmonoester und mindestens eine Fettsäure sowie Verfahren zu deren Herstellung. Weiterer Gegenstand der vorliegenden Erfindung ist die Verwendung entsprechender Zusammensetzungen als Perlglanzmittel bzw. Trübungsmittel in kosmetischen Zusammensetzungen und Waschmitteln.

Perlglanz- und Trübungsmittel werden in kosmetischen Zusammensetzungen und Waschmitteln häufig verwendet, um die Ästhetik entsprechender Zubereitungen zu verbessern und ihnen eine besonders pflegende Erscheinung zu verleihen. Um die hohen Anforderungen des Marktes bezüglich sensorischer Eigenschaften zu erfüllen, werden daher kontinuierlich neue Perlglanz- und Trübungsmittel entwickelt und deren Eignung in kosmetischen Zusammensetzungen und Waschmitteln getestet.

Die derzeit kommerziell erhältlichen bzw. beschriebenen Perlglanzmittel und Trübungsmittel sind bei ihrer Verwendung in kosmetischen Zusammensetzungen und Waschmitteln noch nicht zufriedenstellend und es besteht daher weiterhin Bedarf an der Bereitstellung neuer Inhaltsstoffe, die zum Einsatz in kosmetischen Mitteln oder Waschmitteln als Perlglanzmittel oder Trübungsmittel geeignet sind.

In der Internationalen Patentanmeldung WO2013/041388 werden Isosorbidester mit einem hohen Gehalt an Monoestern und deren vielfältige Verwendung in der Kosmetik beschrieben.

Erfindungsgemäß wurden nun spezielle Zusammensetzungen, die Derivate des Isosorbids enthalten, entwickelt, die vorteilhafterweise in kosmetischen Mitteln oder Waschmitteln eingesetzt werden können.

Demgemäß betrifft die vorliegende Erfindung eine Zusammensetzung, enthaltend
in einer Menge von mindestens 70 Gew.-% eine Mischung von Isosorbiddistearat, Isosorbiddipalmitat und Isosorbidpalmitatstearat als Bestandteil (A),
in einer Menge von 0,01 Gew.-% bis 20 Gew.-% eine Mischung von Isosorbidmonostearat und Isosorbidmonopalmitat als Bestandteil (B) und
in einer Menge von 1 bis 30 Gew.-% eine Mischung von Stearinsäure und Palmitinsäure als Bestandteil (C), bezogen auf die Zusammensetzung.

Erfindungsgemäß wurde herausgefunden, dass eine Zusammensetzung, die mindestens einen Isosorbiddiester, mindestens einen Isosorbidmonoester und mindestens eine Fettsäure enthält, einen verbesserten Perlglanzeffekt und einen verbesserten Trübungseffekt in kosmetischen Zusammensetzungen und Waschmitteln bewirkt.

### Zusammensetzung

Isosorbid (oder 1,4;3,6-Dianhydrosorbitol) ist das Anhydrid des Sorbitols und kommerziell erhältlich. Es kann beispielsweise durch Erwärmen von Sorbitol in Gegenwart von konzentrierter Schwefel- oder Salzsäure erhalten werden. Darüber hinaus kann Isosorbid ausgehend von geeigneten Polysacchariden nach Hydrolyse zu D-Glucose und anschließender Reduktion zu D-Sorbitol durch intramolekulare, zweifache Dehydratisierung erhalten werden. Als Rohstoffquelle wird großtechnisch Stärke oder Cellulose verwendet. Isosorbid ist für Anwendungen im kosmetischen Bereich und im Waschmittelbereich daher ein interessanter Baustein, da er aus nachwachsenden Rohstoffen hergestellt wird.

Durch an sich dem Fachmann bekannte Verfahren können verscheiden Mono- und/oder Diester des Isosorbids erhalten werden.

Die erfindungsgemäß zu verwendenden Isosorbiddiester des Bestandteils (A) weisen die allgemeine Formel (I) auf 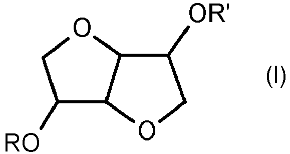 wobei R und R', unabhängig voneinander, jeweils für einen Rest COR" stehen, abgeleitet von einer C₁₆-C₁₈-Fettsäure und ausgewählt wird aus der Gruppe bestehend aus Isosorbiddistearat, Isosorbiddipalmitat und Isosorbidpalmitatstearat.

Die erfindungsgemäß zu verwendenden Isosorbidmonoester des Bestandteils (B) weisen die allgemeine Formel (II) auf 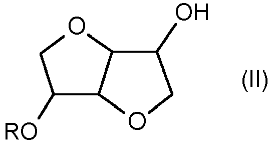 wobei R für einen Rest COR' steht, abgeleitet von einer C₁₆-C₁₈-Fettsäure und ausgewählt wird aus der Gruppe bestehend aus Isosorbidmonostearat und Isosorbidmonopalmitat.

Die oben dargestellten allgemeinen Formeln (I) und (II) umfassen im Rahmen der vorliegenden Erfindung auch alle Stereoisomere des Isosorbids, insbesondere Isoidid und Isomannid, sowie beliebige Mischungen davon. Ferner umfasst die allgemeine Formel (I) auch alle Kombinationen der Reste R und R' untereinander.

Im Rahmen der vorliegenden Erfindung haben sich insbesondere die Diester von Isosorbid mit einer C₁₆- bis C₁₈-Fettsäure als geeignet erwiesen, die gewünschten Perlglanzeigenschaften und Trübungsmitteleigenschaften in kosmetischen Zusammensetzungen und Waschmitteln zu erreichen.

In der erfindungsgemäßen Zusammensetzung handelt es sich bei dem Bestandteil (A) um eine Mischung von Isosorbiddistearat, Isosorbiddipalmitat und Isosorbidpalmitatstearat.

Für die im Rahmen der vorliegenden Erfindung als Bestandteil (A) besonders bevorzugte Mischung aus Isosorbiddistearat und Isosorbiddipalmitat beträgt das Gewichtsverhältnis von Isosorbiddipalmitat zu Isosorbiddistearat in der Zusammensetzung vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98. Bei der Verwendung einer Mischung von Isosorbiddistearat und Isosorbiddipalmitat mit den vorstehend genannten relativen Verhältnissen, d.h. insbesondere mit einem Überschuss an Isosorbiddistearat, sind die Perlglanzeigenschaften und die Trübungsmitteleigenschaften ganz besonders ausgeprägt.

Der Bestandteil (A) ist in der erfindungsgemäßen Zusammensetzung in einer Menge von mindestens 70 Gew.-%, bezogen auf die Zusammensetzung, enthalten.

Bevorzugt ist der Bestandteil (A) in einer Menge von 75 Gew.-% bis 95 Gew.-%, weiter bevorzugt 80 Gew.-% bis 90 Gew.-%, noch weiter bevorzugt 82 Gew.-% bis 88 Gew.-%, jeweils bezogen auf die Zusammensetzung, in der erfindungsgemäßen Zusammensetzung enthalten, um einen guten Perlglanzeffekt und Trübungsmitteleffekt zu erreichen.

### Bestandteil (B) - Isosorbidmonoester

In der erfindungsgemäßen Zusammensetzung ist als Bestandteil (B) eine Mischung von Isosorbidmonostearat und Isosorbidmonopalmitat enthalten.

Für die im Rahmen der vorliegenden Erfindung als Bestandteil (B) diese besonders bevorzugte Mischung aus Isosorbidmonostearat und Isosorbidmonopalmitat beträgt das Gewichtsverhältnis von Isosorbidmonopalmitat zu Isosorbidmonostearat in der Zusammensetzung vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98. Bei der Verwendung einer Mischung von Isosorbidmonostearat und Isosorbidmonopalmitat in den vorstehend genannten relativen Verhältnissen sind die Perlglanzeigenschaften und die Trübungsmitteleigenschaften ganz besonders ausgeprägt.

Der Bestandteil (B) ist in einer Menge von 0,01 Gew.-% bis 20 Gew.-%, weiter bevorzugt 0,5 Gew.-% bis 15 Gew.-%, noch weiter bevorzugt 1 Gew.-% bis 10 Gew.-%, jeweils bezogen auf die Zusammensetzung, in der erfindungsgemäßen Zusammensetzung enthalten, um einen guten Perlglanzeffekt und Trübungsmitteleffekt zu erreichen.

### Bestandteil (C) - Fettsäure

In der erfindungsgemäßen Zusammensetzung wird als Bestandteil (C) eine Fettsäure verwendet. Hierbei handelt es sich um eine Mischung von Stearinsäure und Palmitinsäure.

Für die im Rahmen der vorliegenden Erfindung als Bestandteil (C) enthaltene Mischung aus Stearinsäure und Palmitinsäure beträgt das Gewichtsverhältnis von Palmitinsäure zu Stearinsäure in der Zusammensetzung vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98.

Der Bestandteil (C) ist in einer Menge von 1 Gew.-% bis 30 Gew.-%, weiter bevorzugt 3 Gew.-% bis 25 Gew.-%, noch weiter bevorzugt 5 Gew.-% bis 20 Gew.-%, jeweils bezogen auf die Zusammensetzung, in der erfindungsgemäßen Zusammensetzung enthalten.

### Bevorzugte Ausführungsformen der erfindungsgemäßen Zusammensetzung

In Folgenden werden besonders bevorzugte Ausführungsformen der erfindungsgemäßen Zusammensetzung beschrieben.

In einer ersten besonders bevorzugten Ausführungsform enthält die erfindungsgemäße Zusammensetzung
- eine Mischung von Isosorbiddistearat, Isosorbiddipalmitat und Isosorbidpalmitatstearat als Bestandteil (A) in einer Menge von 75 Gew.-% bis 95 Gew.-%,
- eine Mischung aus Isosorbidmonostearat und Isosorbidmonopalmitat als Bestandteil (B) in einer Menge von 0,01 Gew.-% bis 20 Gew.-%, und
- eine Mischung aus Stearinsäure und Palmitinsäure als Bestandteil (C) in einer Menge von 1 Gew.-% bis 30 Gew.-%, wobei die Mengenangaben jeweils auf die Zusammensetzung bezogen sind.

In einer zweiten besonders bevorzugten Ausführungsform enthält die erfindungsgemäße Zusammensetzung
- eine Mischung von Isosorbiddistearat, Isosorbiddipalmitat und Isosorbidpalmitatstearat als Bestandteil (A) in einer Menge von 80 Gew.-% bis 90 Gew.-%,
- eine Mischung aus Isosorbidmonostearat und Isosorbidmonopalmitat als Bestandteil (B) in einer Menge von 0,5 Gew.-% bis 15 Gew.-%, und
- eine Mischung aus Stearinsäure und Palmitinsäure als Bestandteil (C) in einer Menge von 3 Gew.-% bis 25 Gew.-%, wobei die Mengenangaben jeweils auf die Zusammensetzung bezogen sind.

In einer dritten bevorzugten Ausführungsform enthält die erfindungsgemäße Zusammensetzung
- eine Mischung von Isosorbiddistearat, Isosorbiddipalmitat und Isosorbidpalmitatstearat als Bestandteil (A) in einer Menge von 82 Gew.-% bis 88 Gew.-%,
- eine Mischung aus Isosorbidmonostearat und Isosorbidmonopalmitat als Bestandteil (B) in einer Menge von 1 Gew.-% bis 10 Gew.-%, und
- eine Mischung aus Stearinsäure und Palmitinsäure als Bestandteil (C) in einer Menge von 5 Gew.-% bis 20 Gew.-%, wobei die Mengenangaben jeweils auf die Zusammensetzung bezogen sind.

In den zuvor beschriebenen ersten bis dritten Ausführungsformen sind die erfindungsgemäßen Zusammensetzungen insbesondere dadurch gekennzeichnet, dass es sich bei dem Isosorbiddiester um eine Mischung aus Isosorbiddistearat, Isosorbiddipalmitat und Isosorbidpalmitatstearat, bei dem Isosorbidmonoester um eine Mischung aus Isosorbidmonostearat und Isosorbidmonopalmitat und bei der Fettsäure um eine Mischung aus Stearinsäure und Palmitinsäure handelt.

In den zuvor beschriebenen ersten bis dritten Ausführungsformen sind die erfindungsgemäßen Zusammensetzungen darüber hinaus insbesondere dadurch gekennzeichnet,
- dass das Verhältnis von Isosorbiddipalmitat zu Isosorbiddistearat in der Zusammensetzung 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98, beträgt;
- das Verhältnis Isosorbidmonopalmitat zu Isosorbidmonostearat in der Zusammensetzung 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98, beträgt; und
- das Verhältnis Palmitinsäure zu Stearinsäure in der Zusammensetzung 45 : 55 bis 1 : 99, weiter bevorzugt 40 : 60 bis 1:99, noch weiter bevorzugt 30 : 70 bis 1 : 99, noch weiter bevorzugt 30 : 70 bis 2 : 98, beträgt.

In einer ganz besonders bevorzugten Ausführungsform der vorliegenden Erfindung umfasst die erfindungsgemäße Zusammensetzung
- Isosorbiddistearat, Isosorbiddipalmitat und Isosorbidpalmitatstearat in einer Menge von 82 bis 88 Gew.-%, bezogen auf die Zusammensetzung, mit einem Verhältnis von Isosorbiddipalmitat zu Isosorbiddistearat von 30 : 70 bis 2 : 98;
- Isosorbidmonostearat und Isosorbidmonopalmitat in einer Menge von 1 bis 10 Gew.-% , bezogen auf die Zusammensetzung, mit einem Verhältnis von Isosorbidmonopalmitat zu Isosorbidmonostearat von 30 : 70 bis 2 : 98; und
- Stearinsäure und Palmitinsäure in einer Menge von 5 bis 20 Gew.-%, bezogen auf die Zusammensetzung, mit einem Verhältnis von Palmitinsäure zu Stearinsäure von 30 : 70 bis 2 : 98.

In einer weiteren bevorzugten unabhängigen Ausführungsform der vorliegenden Erfindung ist die erfindungsgemäße Zusammensetzung dadurch gekennzeichnet, dass das Gewichtsverhältnis von Diestern des Isosorbids zu Monoestern des Isosorbids mindestens 4 : 1, weiter bevorzugt mindestens 6 : 1, noch weiter bevorzugt mindestens 8 : 1, noch weiter bevorzugt mindestens 10 : 1, beträgt.

In einer weiteren unabhängigen Ausführungsform der vorliegenden Erfindung ist die erfindungsgemäße Zusammensetzung dadurch gekennzeichnet, dass die Menge an Isosorbiddistearat, Isosorbidmonostearat und Stearinsäure mindestens 70 Gew.-%, weiter bevorzugt mindestens 80 Gew.-%, noch weiter bevorzugt mindestens 90 Gew.-%, beträgt.

Die in der erfindungsgemäßen Zusammensetzung verwendeten Isosorbidester können durch an sich bekannte Veresterungsverfahren synthetisiert werden. In der WO 01/83488 A wird exemplarisch eine geeignete Methode offenbart mit welcher Mono- oder Diester des Isosorbids oder Mischungen aus Mono- und Diestern des Isosorbids erhalten werden können.

Die vorliegende Erfindung betrifft daher auch ein Verfahren zur Herstellung einer erfindungsgemäßen Zusammensetzung, das durch den Verfahrensschritt des Veresterns von Isosorbid mit mindestens einer Fettsäure unter Erhalt eines Veresterungsprodukts gekennzeichnet ist.

Das erfindungsgemäße Verfahren kann dabei in Gegenwart eines Veresterungskatalysators durchgeführt werden, wobei Zinnoxalat einen geeigneten Katalysator darstellt.

Wenn im Rahmen der vorliegenden Erfindung ein Veresterungskatalysator verwendet wird, wird der verwendeten Veresterungskatalysator üblicherweise nach der Veresterungsreaktion deaktiviert, insbesondere wird der verwendete Veresterungskatalysator hydrolysiert.

Im Anschluss an die Veresterungsreaktion und der gegebenenfalls durchzuführenden Deaktivierung des Katalysators wird das resultierende Reaktionsprodukt üblicherweise aufgereinigt, beispielsweise durch Filtration oder Destillation unter Vakuum.

Die Veresterung selbst wird üblicherweise bei einer Temperatur von 160 bis 230 °C, weiter bevorzugt 170 bis 220 °C, noch weiter bevorzugt 180 bis 220 °C, durchgeführt.

Bei der Herstellung der Mono- und Diester des Isosorbids ist zu beachten, dass je nach Überschuss des eingesetzten Isosorbids bzw. der Fettsäure ein unterschiedliches Verhältnis an Mono- und Diester entsteht, da die beiden Hydroxylgruppen durch ihre exo- bzw. endo-Anordnung unterschiedlich reaktiv sind

In dem erfindungsgemäßen Verfahren wird üblicherweise ein Überschuss an Fettsäure zu Isosorbid von mindestens 2,05 Äquivalente Fettsäure bezogen auf 1 Äquivalent Isosorbid verwendet. Besonders bevorzugt ist ein Überschuss an Fettsäure zu Isosorbid von 2,05 bis 2,5 Äquivalente, weiter bevorzugt 2,1 bis 2,4 Äquivalente, noch weiter bevorzugt 2,2 bis 2,3 Äquivalente, jeweils bezogen auf 1 Äquivalent Isosorbid.

Das erfindungsgemäße Verfahren wird üblicherweise mit diesem Überschluss an Fettsäure so lange durchgeführt, bis die erfindungsgemäß definierten Mengen an Bestandteil (A), (B) und (C) in der beanspruchten Zusammensetzung erreicht werden. Dieses ist durch den Fachmann mit üblichen Maßnahmen, beispielsweise mittels GC-Kontrolle und Säurezahlbestimmung, feststellbar. Daher wird die erfindungsgemäße Zusammensetzung vorzugsweise in einer Eintopf-Reaktion hergestellt, bei welcher der Isosorbiddiester und der Isosorbidmonoester ausgehend von Isosorbid und einer oder mehrerer Fettsäuren gleichzeitig gebildet werden. Durch Verwendung eines Überschusses an Fettsäure verbleibt von dieser ebenfalls ein Rest in der erfindungsgemäßen Zusammensetzung. Allerdings ist auch eine Herstellung der erfindungsgemäßen Zusammensetzung durch Mischung der Einzelbestandteile möglich.

### Verwendung in kosmetischen Mitteln

Die erfindungsgemäße Zusammensetzung kann bevorzugt als Perlglanzmittel in kosmetischen Mitteln, insbesondere tensidischen kosmetischen Mitteln, verwendet werden. Bei den kosmetischen Mitteln, insbesondere tensidischen kosmetischen Mitteln, handelt es sich im Allgemeinen um flüssige kosmetische Mittel.

Unter kosmetischen Mitteln sind hier alle dem Fachmann bekannten Mittel zu verstehen, die ausschließlich oder überwiegend dazu bestimmt sind, äußerlich am Körper des Menschen oder in seiner Mundhöhle zur Reinigung, Pflege, zum Schutz, zur Erhaltung eines guten Zustandes, zur Parfümierung, zur Veränderung des Aussehens oder dazu angewendet zu werden, den Körpergeruch zu beeinflussen.

Die erfindungsgemäßen kosmetischen Mittel können im Besonderen Formulierungen zur Körperpflege sein, z. B. eine Körpermilch, Cremes, Lotionen, sprühbare Emulsionen, Produkte zur Eliminierung des Körpergeruchs etc. Die Kohlenwasserstoffe lassen sich auch in tensidhaltigen Formulierungen wie z. B. Schaum- und Duschbädern, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insekten-repellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Als oberflächenaktive Stoffe (Tenside) können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Fettsäureglucamide, Alkylamidobetaine, Amphoacetale und/oder Proteinfettsäurekondensate, letztere vorzugsweise auf Basis von Soja- oder Weizenproteinen.

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 bis 50 Gew.-%, vorzugsweise 5 bis 25 Gew.-% und insbesondere 5 bis 15 Gew.-% enthalten. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbe-henat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen.

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Die kosmetischen Mittel enthalten die erfindungsgemäß beanspruchten Zusammensetzungen als Perlglanzmittel. Allerdings können die kosmetischen Zusammensetzungen auch weitere Perlglanzmittel enthalten. In diesem Sinn kommen als Perlglanzwachse, insbesondere für den Einsatz in tensidischen Formulierungen, beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen (ohne die Sorbitanderivate) mit 2 bis 15 Kohlenstoffatomen und 2 bis 10 Hydroxylgruppen sowie deren Mischungen.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z. B. Magnesium-, Aluminium- und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Im Besonderen sind solche kosmetischen Mittel bevorzugt, die eine wässerige- und eine Ölphase nebeneinander aufweisen und z.B. in Form einer Emulsion (sowohl Wasser-in-ÖI, als auch Öl-in-Wasser) vorliegen und die als einen Bestandteil ein oder mehrere Isosorbid-Derivate gemäß der obigen Definition enthalten. Dabei können die Isosorbid-Derivate als Ölphase bzw. Emollient, oder als Bestandteil der Ölphase eingesetzt werden. Sie können aber, wie im Weiteren noch ausgeführt wird, in Abhängigkeit von ihrer Struktur, auch bestimmte funktionale Eigenschaften vermitteln.

Die erfindungsgemäße Zusammensetzung wird in den kosmetischen Mitteln als Perlglanzmittel vorzugsweise in einer Menge von mindestens 0,1 Gew.-%, bezogen auf das kosmetische Mittel, verwendet.

Bevorzugt wird die erfindungsgemäße Zusammensetzung in dem kosmetischen Mittel in einer Menge von 0,1 bis 12 Gew.-%, weiter bevorzugt 0,5 bis 6 Gew.-%, noch weiter bevorzugt 0,75 bis 3,5 Gew.-%, jeweils bezogen auf das kosmetische Mittel, insbesondere tensidische kosmetische Mittel, verwendet.

### Verwendung in Waschmitteln

Die erfindungsgemäße Zusammensetzung kann darüber hinaus bevorzugt als Trübungsmittel in Waschmitteln, insbesondere tensidischen Waschmitteln, verwendet werden. Bei den Waschmitteln, insbesondere tensidischen Waschmitteln, handelt es sich im Allgemeinen um flüssige Waschmittel.

Entsprechende Waschmittel enthalten anionische und/oder amphothere und/oder nichtionische Tenside sowie Wasser und ggf. weitere für solche Mittel typischen Inhaltsstoffe.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide.

Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugt sind anionische Tenside enthalten und hier insbesondere Alkylethersulfate.

Alkylethersulfate ("Ethersulfate") stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden. Im Sinne der Erfindung kommen Ethersulfate in Betracht, die der Formel (II) folgen,R²O-(CH₂CH₂O)ₘSO₃X (II) in der R² für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}-Kokosfettalkoholfraktionen in Form ihrer Natrium- und/oder Magnesiumsalze.

Die Reinigungsmittel können weiterhin Farbstoffe, Duftstoffe, Perlglanzmittel, Trübungsmittel, Komplexbildner anorganische oder organische Säuren und/oder Basen, Builder, Bleichmittel, Entschäumer, aber auch Polymere (z.B. als Verdicker aber auch als Builder), Hydrotope bzw. Lösungsvermittler und dergleichen enthalten. Vorzugsweise enthalten die Reinigungsmittel Polymere, wobei zu den Details auf die obige Beschreibung hierzu verwiesen wird. Diese Stoffe werden dann üblicherweise in Mengen von insgesamt bis zu 20 Gew.-%, vorzugsweise aber nur bis maximal 15 Gew.-%, insbesondere von 1,5 bis 5 Gew.- %, jeweils bezogen auf das Gesamtgewicht der Reinigungsmittel, eingesetzt.

Der pH-Wert der Reinigungsmittel liegt vorzugsweise im Bereich von 5,0 bis 10,0 vorzugsweise 5,5 bis 8,0. Bevorzugt weisen die Reinigungsmittel einen pH-Wert im Bereich von 6 bis 7,5 auf. Bei sauren Reinigern, wie sie im Badbereich häufig Verwendung finden, sind aber auch deutlich kleinere pH-Wert, typischerweise von 2 bis 5, vorzugsweise von 3,5 bis 4,5 möglich.

Die im Rahmen der vorliegenden Erfindung beschriebenen Waschmittel können insbesondere zur Reinigung von Web-, Strick- und/oder Walkwaren, insbesondere Textilien, Teppiche und/oder Gardinen, eingesetzt werden. Darüber hinaus eignen sich die Waschmittel zur Reinigung von Bedarfsgegenständen. Im Rahmen der vorliegenden Erfindung werden unter dem Begriff der "Bedarfsgegenstände" Gegenstände, die unter die Regelung von § 2 Abs. 6 LFGB zu subsumieren sind, verstanden.

Darüber hinaus eignen sich die erfindungsmäßen Waschmittel zur Reinigung von harten Oberflächen, insbesondere von Metall, Glas, Porzellan, Keramik, Kacheln, Stein, lackierte Oberflächen, Kunststoffen, Holz und/oder Leder, und können in der maschinellen Reinigung eingesetzt werden.

Die erfindungsgemäße Zusammensetzung wird in dem Waschmittel als Trübungsmittel vorzugsweise in einer Menge von mindestens 0,1 Gew.-%, bezogen auf das Waschmittel, verwendet.

Bevorzugt wird die erfindungsgemäße Zusammensetzung in dem Waschmittel in einer Menge von 0,1 bis 12 Gew.-%, weiter bevorzugt 0,5 bis 6 Gew.-%, noch weiter bevorzugt 1 bis 3,5 Gew.-%, jeweils bezogen auf das Waschmitteln, insbesondere tensidische Waschmittel, verwendet.

### Ausführungsbeispiele:

Es wurden die im Folgenden beschriebenen Untersuchungen zu den Eigenschaften der Isosorbid-Derivate vorgenommen. Soweit Inhaltstoffe genannt werden, wurde die INCI-Nomenklatur angewendet.

Die Testsubstanzen wurden in einer kosmetischen Formulierung eingearbeitet.

Der Perlglanz wurde optisch durch Vergleich mit einem Standard, dem Perlglanzmittelwachs (EGDS = Cutina AGS), beurteilt und auf einer Skala von 0 bis 2 bewertet (0 = Perlglanz schlechter als Standard, 1 = Perlglanz vergleichbar mit Standard, 2 = Perlglanz besser als Standard).

| | C-Kettenverteilung C16 und C18 | | GC wt% | | |
|---|---|---|---|---|---|
| | % C16 | % C18 | Monoester | Diester | Fettsäure |
| Probe 1 | 2 | 98 | 6 | 86 | 8 |
| Probe 2 | 30 | 70 | 3 | 89 | 8 |
| Probe 3 | 45 | 55 | 4 | 83 | 13 |
| Probe 4 | 30 | 70 | 33 | 65 | 2 |

**Formulierung 1**

| | Gew.% | | |
|---|---|---|---|
| Testsubstanz Probe 1-4 | 20 | | |
| Comperlan® 100 (INCI:Cocamide MEA) | 2 | | |
| Texapon® N 70 (Kokosfettalkohol+2EO-sulfat-Natriumsalz; 79%ig) | 22 | | |
| Na-benzoat | 0,5 | | |
| Na-chlorid | 1,0 | | |
| Zitronensäure (50%ig) | 0,5 | | |
| Wasser | Ad 100 | | |

| | | | |
|---|---|---|---|
| pH: 4-5 | | | |

Der Perlglanzeffekt (Brillianz) wurde als 5 Gew.-%ige wässrige Verdünnung der Formulierung 1 bestimmt.

| | Perlglanz Effekt der Formulierung 1 mit | |
|---|---|---|
| | | |
| Probe 1 | | 1 |
| Probe 2 | | 2 |
| Probe 3 | | 1-2 |
| Probe 4 | | 0 |

Die Formulierung 1 mit den Proben 1 bis 4 zeigen alle einen Perlglanz. Die Formulierung 1 mit der Probe 1 bis 3 zeigt einen besseren oder gleichwertigen Perlglanz wie eine Formulierung mit der äquivalenten Menge an Standard-Perlglanzmittel (EGDS = Cutina AGS). Die Formulierung 1 mit der Probe zeigt absolut den besten Perlglanz, der auch deutlich besser ist als der Standard.

## Patentansprüche

1. Zusammensetzung, enthaltend
in einer Menge von mindestens 70 Gew.-% eine Mischung von Isosorbiddistearat, Isosorbiddipalmitat und Isosorbidpalmitatstearat als Bestandteil (A),
in einer Menge von 0,01 Gew.-% bis 20 Gew.-% eine Mischung von Isosorbidmonostearat und Isosorbidmonopalmitat als Bestandteil (B) und
in einer Menge von 1 bis 30 Gew.-% eine Mischung von Stearinsäure und Palmitinsäure als Bestandteil (C), bezogen auf die Zusammensetzung.

2. Zusammensetzung nach Anspruch 1, **dadurch gekennzeichnet, dass** das Gewichtsverhältnis von Isosorbiddipalmitat zu Isosorbiddistearat in der Zusammensetzung 45 : 55 bis 1 : 99 beträgt, das Gewichtsverhältnis von Isosorbidmonopalmitat zu Isosorbidmonostearat in der Zusammensetzung 45 : 55 bis 1 : 99 beträgt und das Gewichtsverhältnis von Palmitinsäure zu Stearinsäure in der Zusammensetzung 45 : 55 bis 1 : 99 beträgt.

3. Zusammensetzung nach einem der Ansprüche 1 bis 2, **dadurch gekennzeichnet, dass** die Zusammensetzung umfasst:
- Isosorbiddistearat, Isosorbiddipalmitat und Isosorbidpalmitatstearat in einer Menge von 82 bis 88 Gew.-%, bezogen auf die Zusammensetzung, mit einem Verhältnis von Isosorbiddipalmitat zu Isosorbiddistearat von 30 : 70 bis 2 : 98;
- Isosorbidmonostearat und Isosorbidmonopalmitat in einer Menge von 1 bis 10 Gew.-%, bezogen auf die Zusammensetzung, mit einem Verhältnis von Isosorbidmonopalmitat zu Isosorbidmonostearat von 30 : 70 bis 2 : 98; und
- Stearinsäure und Palmitinsäure in einer Menge von 5 bis 20 Gew.-%, bezogen auf die Zusammensetzung, mit einem Verhältnis von Palmitinsäure zu Stearinsäure von 30 : 70 bis 2 : 98.

4. Zusammensetzung nach einem der Ansprüche 1 bis 3, **dadurch gekennzeichnet, dass** das Gewichtsverhältnis von Diestern des Isosorbids zu Monoestern des Isosorbids mindestens 4 : 1 beträgt.

5. Zusammensetzung nach einem der Ansprüche 1 bis 4, **dadurch gekennzeichnet, dass** die Menge an Isosorbiddistearat, Isosorbidmonostearat und Stearinsäure mindestens 70 Gew.-%, bezogen auf die Zusammensetzung, beträgt.

6. Verfahren zur Herstellung einer Zusammensetzung gemäß einem der Ansprüche 1 bis 5, **gekennzeichnet durch** den Verfahrensschritt des Veresterns von Isosorbid mit mindestens einer Fettsäure unter Erhalt eines Veresterungsprodukts.

7. Verfahren nach Anspruch 6, **dadurch gekennzeichnet, dass** bei der Veresterung ein Überschuss an Fettsäure zu Isosorbid von mindestens 2,05 Äquivalenten Fettsäure bezogen auf 1 Äquivalent Isosorbid verwendet wird.

8. Verwendung der Zusammensetzung gemäß einem der Ansprüche 1 bis 5 als Perlglanzmittel und/oder Trübungsmittel.

9. Verwendung der Zusammensetzung gemäß Anspruch 8 in kosmetischen Zusammensetzungen oder Waschmitteln, insbesondere in Handwaschmitteln oder in Wollwaschmitteln, insbesondere zur Reinigung von Web-, Strick- und/oder Walkwaren, insbesondere Textilien, Teppiche und/oder Gardinen, und zur Reinigung von Bedarfsgegenständen.

10. Verwendung nach Anspruch 9 zur Reinigung von harten Oberflächen, insbesondere von Metall, Glas, Porzellan, Keramik, Kacheln, Stein, lackierte Oberflächen, Kunststoffen, Holz und/oder Leder.

## Claims

1. A composition comprising
a mixture of isosorbide distearate, isosorbide dipalmitate and isosorbide palmitate stearate in an amount of at least 70% by weight as constituent (A),
a mixture of isosorbide monostearate and isosorbide monopalmitate in an amount of 0.01% by weight to 20% by weight as constituent (B) and
a mixture of stearic acid and palmitic acid in an amount of 1% to 30% by weight as constituent (C), based on the composition.

2. The composition according to claim 1, wherein the weight ratio of isosorbide dipalmitate to isosorbide distearate in the composition is 45:55 to 1:99, the weight ratio of isosorbide monopalmitate to isosorbide monostearate in the composition is 45:55 to 1:99 and the weight ratio of palmitic acid to stearic acid in the composition is 45:55 to 1:99.

3. The composition according to either of claims 1 and 2, the composition comprising:
- isosorbide distearate, isosorbide dipalmitate and isosorbide palmitate stearate in an amount of 82 to 88% by weight, based on the composition, with a ratio of isosorbide dipalmitate to isosorbide distearate of 30:70 to 2:98;
- isosorbide monostearate and isosorbide monopalmitate in an amount of 1 to 10% by weight, based on the composition, with a ratio of isosorbide monopalmitate to isosorbide monostearate of 30:70 to 2:98; and
- stearic acid and palmitic acid in an amount of 5 to 20% by weight, based on the composition, with a ratio of palmitic acid to stearic acid of 30:70 to 2:98.

4. The composition according to any of claims 1 to 3, wherein the weight ratio of diesters of the isosorbide to monoesters of the isosorbide is at least 4:1.

5. The composition according to any of claims 1 to 4, wherein the amount of isosorbide distearate, isosorbide monostearate and stearic acid is at least 70% by weight, based on the composition.

6. A process for the preparation of a composition according to any of claims 1 to 5, wherein an esterification product is obtained by the process stage of the esterification of isosorbide with at least one fatty acid.

7. The process according to claim 6, wherein use is made, in the esterification, of an excess of fatty acid to isosorbide of at least 2.05 equivalents of fatty acid, based on 1 equivalent of isosorbide.

8. The use of the composition according to any of claims 1 to 5 as pearlizing agent and/or opacifier.

9. The use of the composition according to claim 8 in cosmetic compositions or detergents, in particular in hand cleansers or in wool detergents, in particular for the cleaning of woven goods, knitwear and/or fulled goods, in particular textiles, carpets and/or curtains, and for the cleaning of necessary articles.

10. The use according to claim 9 for the cleaning of hard surfaces, in particular of metal, glass, porcelain, ceramic, tiles, stone, varnished surfaces, plastics, wood and/or leather.

## Revendications

1. Composition, contenant
en une quantité d'au moins 70 % en poids, un mélange de distéarate d'isosorbide, de dipalmitate d'isosorbide et de palmitate stéarate d'isosorbide en tant qu'ingrédient (A),
en une quantité de 0,01 % en poids à 20 % en poids, un mélange de monostéarate d'isosorbide, et de monopalmitate d'isosorbide en tant qu'ingrédient (B) et
en une quantité de 1 à 30 % en poids, un mélange d'acide stéarique et d'acide palmitique en tant qu'ingrédient (C), par rapport à la composition.

2. Composition selon la revendication 1, **caractérisée en ce que** le rapport pondéral de dipalmitate d'isosorbide à distéarate d'isosorbide dans la composition est de 45:55 à 1:99, le rapport pondéral de monopalmitate d'isosorbide à monostéarate d'isosorbide dans la composition est de 45:55 à 1:99 et le rapport pondéral d'acide palmitique à acide stéarique dans la composition est de 45:55 à 1:99.

3. Composition selon l'une quelconque des revendications 1 et 2, **caractérisée en ce que** la composition comprend :
- du distéarate d'isosorbide, du dipalmitate d'isosorbide et du palmitate stéarate d'isosorbide en une quantité de 82 à 88 % en poids, par rapport à la composition, avec un rapport de dipalmitate d'isosorbide à distéarate d'isosorbide de 30:70 à 2:98 ;
- du monostéarate d'isosorbide et du monopalmitate d'isosorbide en une quantité de 1 à 10 % en poids, par rapport à la composition, avec un rapport de monopalmitate d'isosorbide à monostéarate d'isosorbide de 30:70 à 2:98 ; et
- de l'acide stéarique et de l'acide palmitique en une quantité de 5 à 20 % en poids, par rapport à la composition, avec un rapport d'acide palmitique à acide stéarique de 30:70 à 2:98.

4. Composition selon l'une quelconque des revendications 1 à 3, **caractérisée en ce que** le rapport pondéral des diesters de l'isosorbide aux monoesters de l'isosorbide est d'au moins 4:1.

5. Composition selon l'une quelconque des revendications 1 à 4, **caractérisée en ce que** la quantité de distéarate d'isosorbide, de monostéarate d'isosorbide et d'acide stéarique est d'au moins 70 % en poids, par rapport à la composition.

6. Procédé pour la préparation d'une composition selon l'une quelconque des revendications 1 à 5, **caractérisé par** l'étape de procédé d'estérification d'isosorbide avec au moins un acide gras avec obtention d'un produit d'estérification.

7. Procédé selon la revendication 6, **caractérisé en ce que** dans l'estérification, on utilise un excès d'acide gras par rapport à l'isosorbide d'au moins 2,05 équivalents d'acide gras par rapport à 1 équivalent d'isosorbide.

8. Utilisation de la composition selon l'une quelconque des revendications 1 à 5 en tant qu'agent nacrant et/ou en tant qu'agent opacifiant.

9. Utilisation de la composition selon la revendication 8 dans des compositions cosmétiques ou des produits de lavage, en particulier dans des produits de lavage des mains ou dans des produits de lavage de la laine, en particulier pour le nettoyage de tissus tissés, de tricots et/ou de tissus foulés, en particulier de textiles, de tapis et/ou de rideaux, et pour le nettoyage d'objets usuels.

10. Utilisation selon la revendication 9 pour le nettoyage de surfaces dures, en particulier de métal, de verre, de porcelaine, de céramique, de carreaux, de pierre, de surfaces laquées, de plastiques, de bois et/ou de cuir.
